# EUROPÄISCHE PATENTSCHRIFT

(11) **EP 0 717 043 B1**
(45) Veröffentlichungstag und Bekanntmachung des Hinweises auf die Patenterteilung: **10.11.1999**
(21) Anmeldenummer: 95119052.9
(22) Anmeldetag: 04.12.1995
(51) Int. Cl.: C07D 471/04, A61K 31/435

(54) **Verwendung von 1,2-überbrückten 1,4-Dihydropyridinen als selektive Kaliumkanalmodulatoren**
Use of 1,2-bridged 1,4-dihydropyridines as selective potassium channel modulators
Utilisation de 1,4-dihydropyridines 1,2-pontées comme un modulateur des canaux de potassium

(30) Priorität: 16.12.1994 DE 4444860
(43) Veröffentlichungstag der Anmeldung: 19.06.1996
(60) Teilanmeldung: 99105350.5
(73) Patentinhaber: BAYER AG, 51368 Leverkusen (DE)
(72) Erfinder: Urbahns, Klaus, Dr., D-42115 Wuppertal (DE); Heine, Hans-Georg, Dr., D-47800 Krefeld (DE); Junge, Bodo, Dr., D-42399 Wuppertal (DE); Glaser, Thomas, Dr., D-51491 Overath (DE); Wittka, Reilinde, D-51069 Köln (DE); De Vry, Jean-Marie-Viktor, Dr., D-51503 Rösrath (DE); Sommermeyer, Henning, Dr., D-51061 Köln (DE)

(56) Entgegenhaltungen:
- DE-A- 2 210 633
- JUSTUS LIEBIGS ANN. CHEM. (JLACBF,00754617);77; (11-12); PP.1888-94, BAYER A.-G.;CHEM.-WISS. LAB. PHARMA; WUPPERTAL; GER., MEYER H ET AL 'Dihydropyridines, II. Synthesis of 1,4-dihydropyridines with bridgehead nitrogen'
- PFLÜGERS ARCHIV-EUROPEAN JOURNAL OF PHYSIOLOGY, Bd. 429, Nr. 2, 1994 Seiten 176-182, FAGNI ET AL. 'Inhibitory effects of dihydropyridines ...'
- BR. J. PHARMACOL, Bd. 111, Nr. 3, 1994 Seiten 903-905, ELLORY ET AL. 'Specific inhibition of Ca-activated K channels ...'
- TRIGGLE ER AL.: "Ca2+ Channel ligands: Structure and Function relationships of the 1,4-dihydropyridines", MED.RES.REV., , , Band 9, Nr. 2, Seiten 123 - 180

## Beschreibung

Die vorliegende Erfindung betrifft neue 1,2-überbrückte 1,4-Dihydropyridine, Verfahren zu ihrer Herstellung und ihre Verwendung als Arzneimittel, als selektive Kaliumkanalmodulatoren, insbesondere zur Behandlung des zentralen Nervensystems.

Aus der Publikation Justus Liebigs Ann. Chem. (1977), 11-12, 1888-94 sind 1,2-überbrückten 1,4-Dihydropyridin-3,5-dicarbonsäureester bekannt.

Außerdem sind 1,2-Hexa- und 1,2-Pentamethylen-1,4-dihydropyridinderivate mit Kreislaufwirkung beschrieben [vgl. US 3,951,988; US 3,935,220 und DE 22 10 633].

Br. J. Pharmacol. 1994, 111, 903 - 905 und Pflügers Archiv-Eur. J. Physiol. 1994, 429, 176- 182 beschreiben Kalziumkanalblocker mit monocyclischer Dihydropyridinstruktur, die gleichzeitig einen Kalziumkanal und Kaliumkanal inhibieren.

Es wurde gefunden, daß 1,2-überbrückte 1,4-Dihydropyridine der allgemeinen Formel (I) 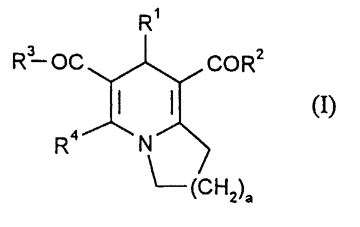 und deren Salze, mit den in der folgenden Tabelle angegebenen Substituentenbedeutungen

| R¹ | R² | a | R³ | R³+R⁴ | R⁴ | Bsp. Nr. |
|---|---|---|---|---|---|---|
| m-NO₂-C₆H₄ | OCH₃ | 2 | - | -CH₂-CH₂-CH₂- | - | 10 |
| p-Cl-C₆H₄ | OCH₃ | 2 | - | -CH₂-CH₂-CH₂- | - | 11 |
| o,m-Cl-C₆H₃ | OCH₃ | 2 | - | -CH₂-CH₂-CH₂- | - | 12 |
| p-Cl-C₆H₄ | OCH₃ | 1 | - | -CH₂-CH₂-CH₂- | - | 13 |
| o,m-Cl-C₆H₃ | OCH₃ | 1 | - | -CH₂-CH₂-CH₂- | - | 14 |
| p-Cl-C₆H₄ | OCH₃ | 1 | - | -CH₂-C(CH₃)₂-CH₂- | - | 15 |
| o,m-Cl-C₆H₃ | OCH₃ | 1 | - | -CH₂-C(CH₃)₂-CH₂- | - | 16 |
| m-NO₂-C₆H₄ | OCH₃ | 1 | - | -CH₂-CH₂-CH₂- | - | 17 |
| o,m-Cl-C₆H₃ | OCH₃ | 1 | -CH₃ | - | CH₃ | 18 |
| p-Cl-C₆H₄ | OCH₃ | 1 | -CH₃ | - | CH₃ | 19 |
| p-NO₂-C₆H₄ | CH₃ | 1 | CH₃ | - | CH₃ | 5 |
| m,p-Cl-C₆H₃ | CH₃ | 1 | CH₃ | - | CH₃ | 6 |
| o,m-Cl-C₆H₃ | CH₃ | 1 | CH₃ | - | CH₃ | 7 |
| p-CF₃-C₆H₄ | CH₃ | 1 | CH₃ | - | CH₃ | 8 |
| m-CF₃,p-Cl-C₆H₃ | CH₃ | 1 | CH₃ | - | CH₃ | 9 |
| o,m-Cl-C₆H₃ | OCH₃ | 1 | OCH₃ | - | CH₃ | 1 |
| p-CF₃-C₆H₄ | OCH₃ | 1 | OCH₃ | - | CH₃ | 2 |
| p-Cl-C₆H₄ | OCH₃ | 2 | OCH₃ | - | CH₃ | 3 |
| p-Cl-C₆H₄ | OCH₃ | 1 | OCH₃ | - | CH₃ | 4 |

überraschenderweise eine selektive modulierende Wirkung auf Kaliumkanäle besitzen und geeignet sind zur Verwendung bei der Bekämpfung von Erkrankungen des zentralen Nervensystems und der Sichelzellenanämie.

Die erfindungsgemäßen Verbindungen können in stereoisomeren Formen existieren, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen.

Die erfindungsgemäßen Verbindungen der allgemeinen Formel (I) zeigen ein nicht vorhersehbares, wertvolles pharmakologisches Wirkspektrum.

Sie sind Kanalmodulatoren mit einer überraschenden Selektivität für calciumabhängige Kalium-Kanäle großer Leitfähigkeit (BK(Ca)-Kanäle), insbesondere die Kalium-Kanäle des zentralen Nervensystems.

Aufgrund ihrer pharmakologischen Eigenschaften können sie für die Herstellung von Arzneimitteln zur Behandlung von zentral degenerativen Erkrankungen eingesetzt werden, wie z.B. bei Auftreten von Demenzen (Multiinfarktdemenz (MID), primär degenerativer Demenz (PDD), prä- und seniler Alzheimerscher Krankheit, HIV-Demenz und andere Demenzformen), Parkinsonscher Krankheit oder amyotrophischer Lateralsklerose sowie multipler Sklerose.

Weiterhin eignen sich die Wirkstoffe zur Behandlung von Hirnleistungsstörungen im Alter, des hirnorganischen Psychosyndroms (HOPS, Organic Brain Syndrom, OBS) und von altersbedingten Gedächtnisstörungen (age associated memory impairment, AAMI).

Sie sind geeignet zur Prophylaxe, Behandlung und zur Bekämpfung der Folgen cerebraler Durchblutungsstörungen wie cerebraler Ischämien, Schlaganfällen, Schädel-Hirn-Traumata und von Subarachnoidalblutungen.

Sie sind wertvoll zur Behandlung von Depressionen und Psychosen, z.B. Schizophrenie. Außerdem eignen sie sich zur Behandlung von Störungen der neuroendokrinen Sekretion sowie der Neurotransmittersekretion und damit zusammenhängenden gesundheitlichen Störungen wie Manie, Alkoholismus, Drogenmißbrauch, Sucht oder krankhaftem Eßverhalten. Weitere Anwendungsgebiete sind die Behandlung von Migräne, Schlafstörungen und von Neuropathien. Darüber hinaus sind sie als Schmerzmittel geeignet.

Die Wirkstoffe sind ferner geeignet zur Behandlung von Störungen des Immunsystems, insbesondere der T-Lymphocyten-Proliferation und zur Beeinflussung der glatten Muskulatur, insbesondere von Uterus, Harnblase und Bronchialtrakt und zur Behandlung damit zusammenhängender Krankheiten wie z.B. Asthma und urinärer Inkontinenz und zur Behandlung von Arrhythmie, Angina und Diabetes.

Die neuen und bekannten erfindungsgemäßen Verbindungen der Formel (I) können hergestellt werden, in dem man
(A) im Fall, daß R³ und R⁴ jeweils für einen offenkettigen Rest stehen, Aldehyde der allgemeinen Formel (II)

   R¹-CHO (II)

   in welcher
   R¹ die oben angegebene Bedeutung hat,
      zunächst durch Umsetzung mit Dioxoverbindungen der allgemeinen Formel (III) 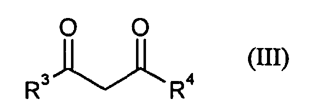 in welcher
   R³ und R⁴ die oben angegebene Bedeutung haben,
      in inerten Lösemitteln, gegebenenfalls in Anwesenheit einer Base,
      in die Benzylidenverbindungen der allgemeinen Formel (IV) 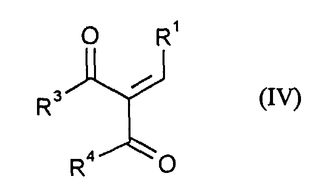 in welcher
   R¹, R³ und R⁴ die oben angegebene Bedeutung haben,
      überführt, welche gegebenenfalls isoliert werden,
   und in einem zweiten Schritt mit Aminen der allgemeinen Formel (V) 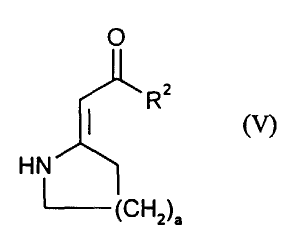 in welcher
   R² und a die oben angegebene Bedeutung haben,
      in inerten Lösemitteln umsetzt, oder
(B) im Fall, daß R³ und R⁴ gemeinsam einen der oben aufgeführten Ringe bilden,
   die Aldehyde der allgemeinen Formel (II) mit Verbindungen der allgemeinen Formel (VI) 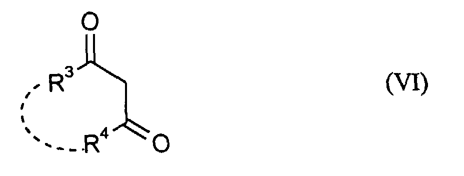 in welcher
   R³ und R⁴ gemeinsam für einen Rest der Formel -CH₂-CH₂-CH₂- oder -CH₂-C(CH₃)₂-CH₂ stehen,
   und den cyclischen Aminen der allgemeinen Formel (V), in einem Lösemittel, gegebenenfalls in Anwesenheit einer Base umsetzt.

Die erfindungsgemäßen Verfahren können durch folgendes Formelschema beispielhaft erläutert werden:

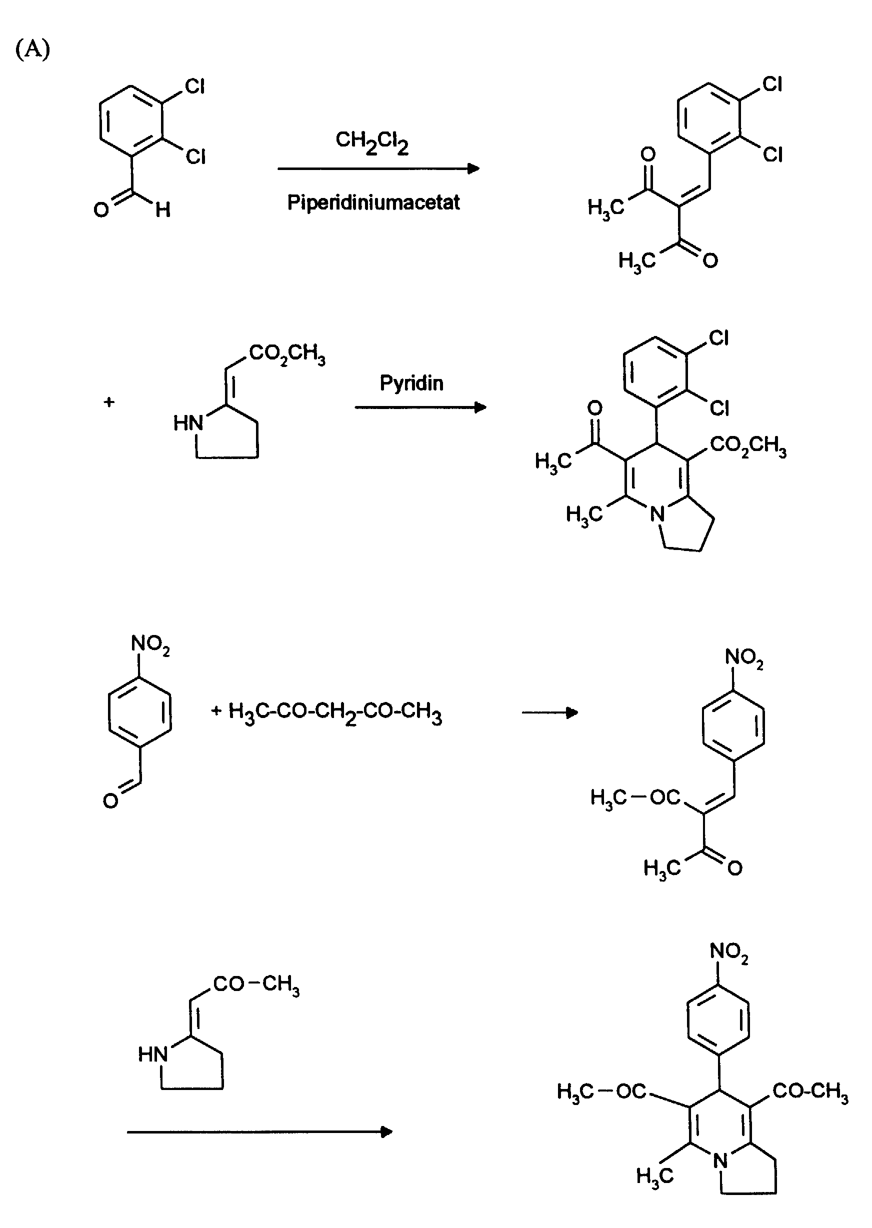

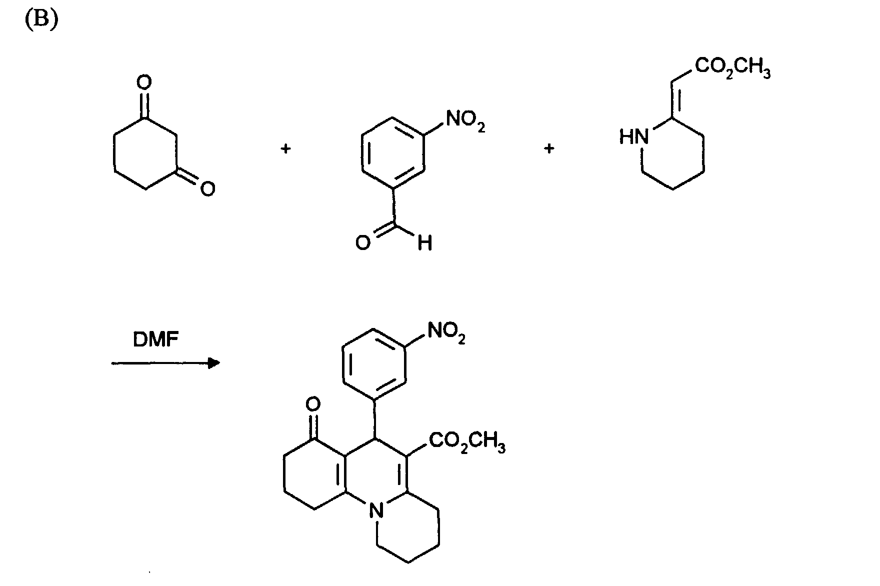

Als Lösemittel für das Verfahren (A) eignen sich im allgemeinen für den ersten und zweiten Schritt des Verfahrens alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether oder Diethylenglykoldimethylether, Acetonitril, Aceton oder Amide wie Hexamethylphosphorsäuretriamid oder Dimethylformamid, oder halogenierte Kohlenwasserstoffe wie Methylenchlorid, Tetrachlorkohlenstoff oder Kohlenwasserstoffe wie Benzol oder Toluol, Pyridin oder Essigsäure. Bevorzugt sind für den ersten Schritt Methylenchlorid und Pyridin oder DMF für den zweiten Schritt.

Als Base für den ersten Schritt eignen sich im allgemeinen Alkalicarbonate oder -alkoholate, wie beispielsweise Kaliumcarbonat oder Kalium-tert.-butylat oder cyclische Amine, wie beispielsweise Piperidin oder Dimethylaminopyridin oder Pyridin, oder C₁-C₄-Alkylamine, wie beispielsweise Triethylamin.

Bei der Durchführung der erfindungsgemäßen Verfahren ist das Verhältnis der an der Reaktion beteiligten Stoffe beliebig. Im allgemeinen arbeitet man jedoch bei molaren Mengen der Reaktanden.

Die Reaktionstemperaturen können in einem größeren Bereich variiert werden. Im allgemeinen arbeitet man zwischen +10°C und +200°C, vorzugsweise zwischen +20°C und +100°C, insbesondere bei der Siedetemperatur des jeweiligen Lösemittels.

Die Umsetzungen können bei Normaldruck, aber auch bei erhöhtem oder erniedrigtem Druck (z.B. 0,5 bis 3 bar) durchgeführt werden. Im allgemeinen arbeitet man bei Normaldruck.

Als Lösemittel für das Verfahren (B) eignen sich ebenfalls die oben aufgeführten Lösemittel. Bevorzugt ist Dimethylformamid oder Pyridin.

Die Reaktionstemperaturen können in einem größeren Bereich variiert werden. Im allgemeinen arbeitet man zwischen +10°C und +200°C, vorzugsweise zwischen +20°C und +150°C, insbesondere bei der Siedetemperatur des jeweiligen Lösemittels.

Enantiomerenreine Formen erhält man z.B., indem man Diastereomerengemische der Verbindungen der allgemeinen Formel (I), in welcher R² oder R³ für einen optisch aktiven Esterrest steht, nach üblicher Methode trennt, anschließend entweder direkt umestert oder zuerst die chiralen Carbonsäuren herstellt und dann durch Veresterung die enantiomerenreinen Dihydropyridine herstellt.

Die Trennung der Diastereomeren erfolgt im allgemeinen entweder durch fraktionierte Kristallisation, durch Säulenchromatographie oder durch Craig-Verteilung. Welches das optimale Verfahren ist, muß von Fall zu Fall entschieden werden, manchmal ist es auch zweckmäßig, Kombinationen der einzelnen Verfahren zu benutzen. Besonders geeignet ist die Trennung durch Kristallisation oder Craig-Verteilung bzw. eine Kombination beider Verfahren.

Die enantiomerenreinen Verbindungen sind auch zugänglich durch Chromatographie der racemischen Ester auf chiralen Phasen.

Die Verbindungen der allgemeinen Formeln (II), (III), (IV), (V) und (VI) sind an sich bekannt oder können nach üblichen Methoden hergestellt werden.

Die erfindungsgemäßen Verbindungen der allgemeinen Formel (I) zeigen ein nicht vorhersehbares Wirkspektrum, insbesondere auf Grund ihrer Selektivität auf calciumabhängige Kalium-Kanäle großer Leitfähigkeit.

### ⁸⁶Rubidium-Efflux aus C6-BU1-Glioma-Zellen

Die Versuche wurden mit geringfügigen Veränderungen entsprechend der von Tas et al. (Neurosci. Lett. 94, 279-284, (1988)) beschriebenen Methode durchgeführt. Dazu werden Ratten C6-BU1-Glioma-Zellen verwendet. Aus den durch Flüssigszintillation erhaltenen Daten wird die durch Ionomycin hervorgerufene Erhöhung des Effluxes über den Basalefflux berechnet und als 100 % gesetzt. Die Stimulationen in Gegenwart von Prüfsubstanzen werden dann auf diesen Wert bezogen.

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formel (I) enthalten, oder die aus einem oder mehreren Wirkstoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-% der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formel (I) in Gesamtmengen von etwa 0,01 bis etwa 100 mg/kg, bevorzugt in Gesamtmengen von etwa 1 mg/kg bis 50 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

### Ausgangsverbindungen

### Beispiel A

2-Acetyl-3-(2,3-dichlorphenyl)-acrylsäuremethylester 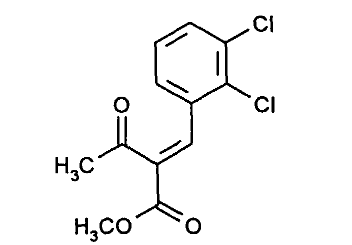 17,5 g (100 mmol) 2,3-Dichlorbenzaldehyd und 11,6 g (100 mmol) Acetessigsäuremethylester werden in 350 ml CH₂Cl₂ mit 1 ml Piperidin und 0,5 ml HOAc 3h am Wasserabscheider gekocht. Danach wird der Ansatz zweimal mit Wasser gewaschen, über MgSO₄ getrocknet und eingeengt. Der Rückstand kristallisiert aus Petrolether / Ether.
Ausbeute. 15,0 g (55%)

### Beispiel B

3-(4-Nitrobenzyliden)-pentan-2,4-dion 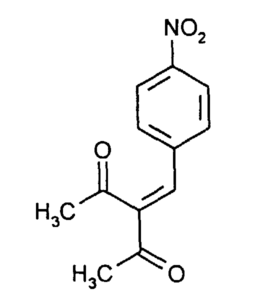 30,2 g (0,2 mol) 4-Nitrobenzaldehyd und 30,0 g (0,3 mol) Acetylaceton werden in 200 ml Isopropanol gelöst und mit 1,2 ml Piperidin sowie 1 ml Eisessig versetzt. Man erwärmt im Wasserbad bis sich eine klare Lösung bildet und rührt anschließend 4 h bei RT. Das Produkt fällt aus und wird abgesaugt. Nach Waschen mit Isopropanol und Ether erhält man 39,3 g (84 %) der Titelverbindung.

### Herstellungsbeispiele

### Beispiel 1

7-(2,3-Dichlorphenyl)-5-methyl-1,2,3,7-tetrahydroindolizin-6,8-dicarbonsäuredimethylester 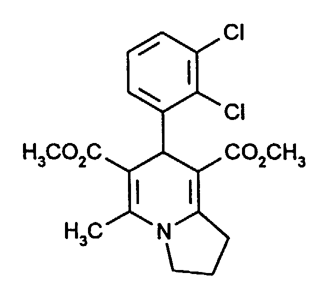 2,7 g (10 mmol) 2-Acetyl-3-(2,3-dichlorphenyl)acrylsäuremethylester und 1,4 g (10 mmol) Pyrrolidin-2-yliden-essigsäuremethylester werden 12 h in 50 ml Isopropanol zum Rückfluß erhitzt. Dazu wird die Reaktionsmischung auf RT abgekühlt und mit 20 ml Petrolether versetzt. Der ausgefallene Niederschlag wird abgesaugt und aus Isopropanol umkristallisiert. Man erhält 1,5 g (37%) der Titelverbindung. MS: 395
R_{f} = 0,33 (PE / AcOEt = 1:1)
m.p.: 173°C

In Analogie zur Vorschrift des Beispiels 1 werden die in der Tabelle 1 aufgeführten Verbindungen, gegebenenfalls im Fall a = 2 unter Verwendung von Piperidin-2-yliden-essigsäuremethylester hergestellt.

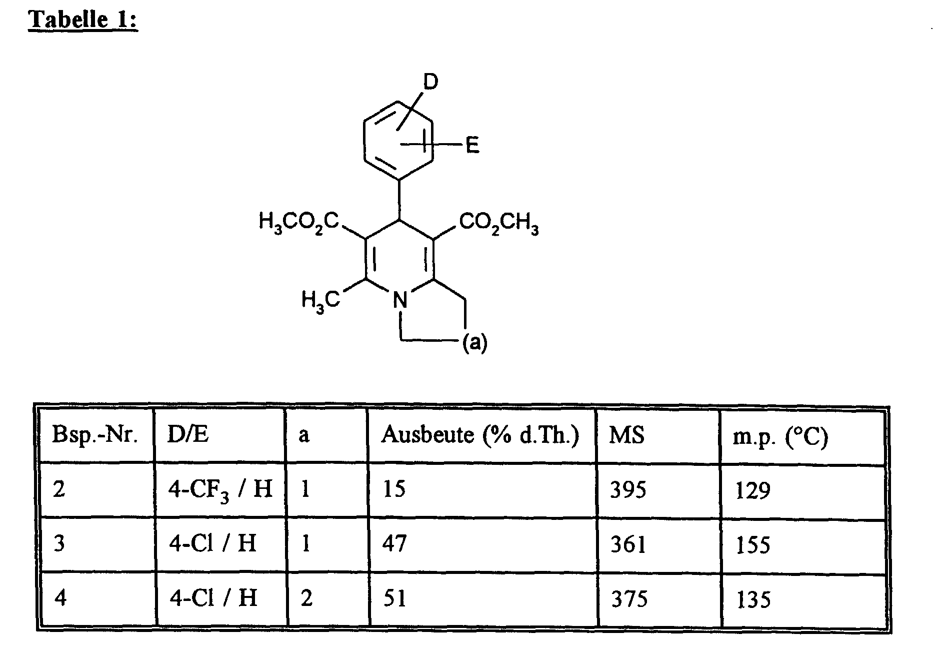

### Beispiel 5

1 -[6-Acetyl-5-methyl-7-(4-nitrophenyl)-1,2,3,7-tetrahydroindolizin-8-yl]ethanon 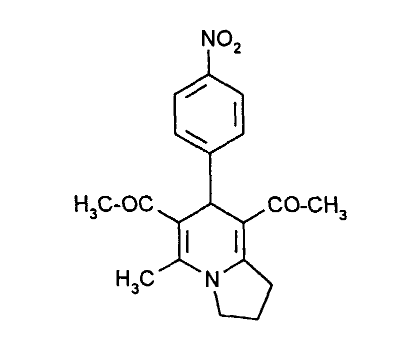 4,66 g (20 mmol) der Verbindung aus Beispiel B und 2,50 g (20 mmol) 1-Pyrrolidin-2-yliden-propan-2-on werden in 80 ml Pyridin 20 h bei 100°C gerührt. Dann engt man ein und reinigt chromatographisch (CH₂Cl₂/AcOEt = 10+1). Aus AcOEt kristallisieren 4,6 g (75 %) der Titelverbindung.
MS: 340
R_{f} = 0,16 (PE/AcOEt = 1+1)

In Analogie zur Vorschrift des Beispiels 5 werden die in der Tabelle 2 aufgeführten Verbindungen hergestellt:

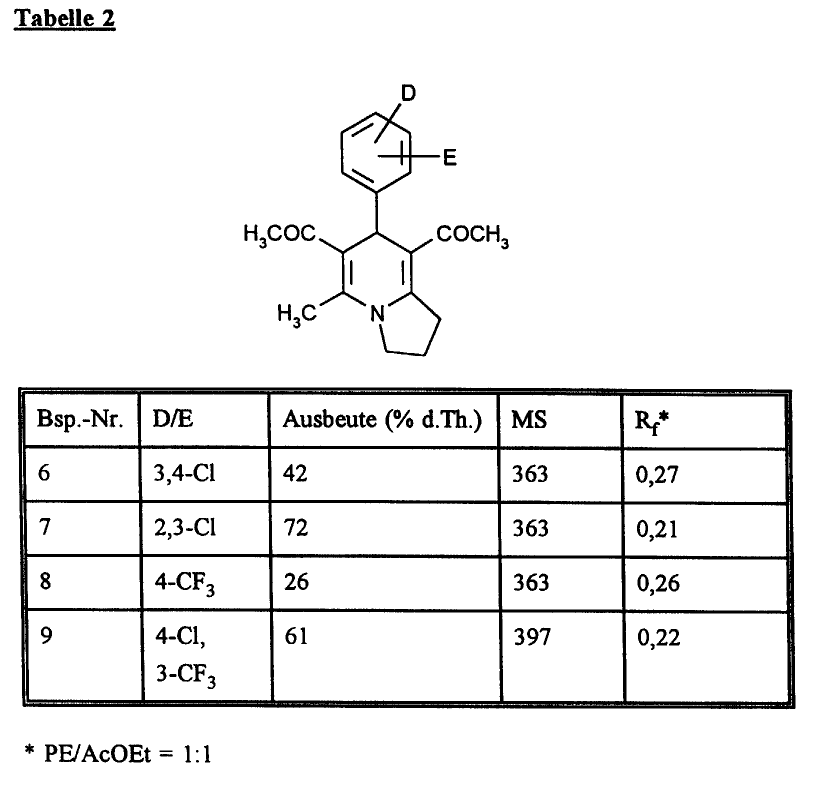

### Beispiel 10

6-(3-Nitrophenyl)-7-oxo-1,2,3,4,6,7,8,9,10-nonahydro-pyrido[1,2-a]quinolin-5-carbonsäuremethylester 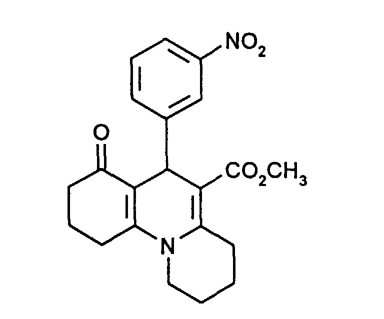 4,28 g (28,3 mmol) 3-Nitrobenzaldehyd, 3,17 g (28,3 mmol) Cyclohexan-1,3-dion und 4,5 g (28,3 mmol) Piperidin-2-yliden-essigsäuremethylester werden in 80 ml DMF am Rückfluß gehalten. Nach 4 h wird eingeengt, zweimal mit Toluol kodestilliert und über Kieselgel gereinigt (Petrolether/CH₂Cl₂ = 2:1). Die entsprechenden Fraktionen werden eingeengt und aus Ether kristallisiert. Man erhält 2,1 g der Titelverbindung.
MS: 382
R_{f} = 0,23

In Analogie zur Vorschrift des Beispiels 10 im Fall a = 1, gegebenenfalls unter Verwendung von Pyrrolidin-2-essigsäuremethylester, werden die in der Tabelle 3 aufgeführten Verbindungen hergestellt:

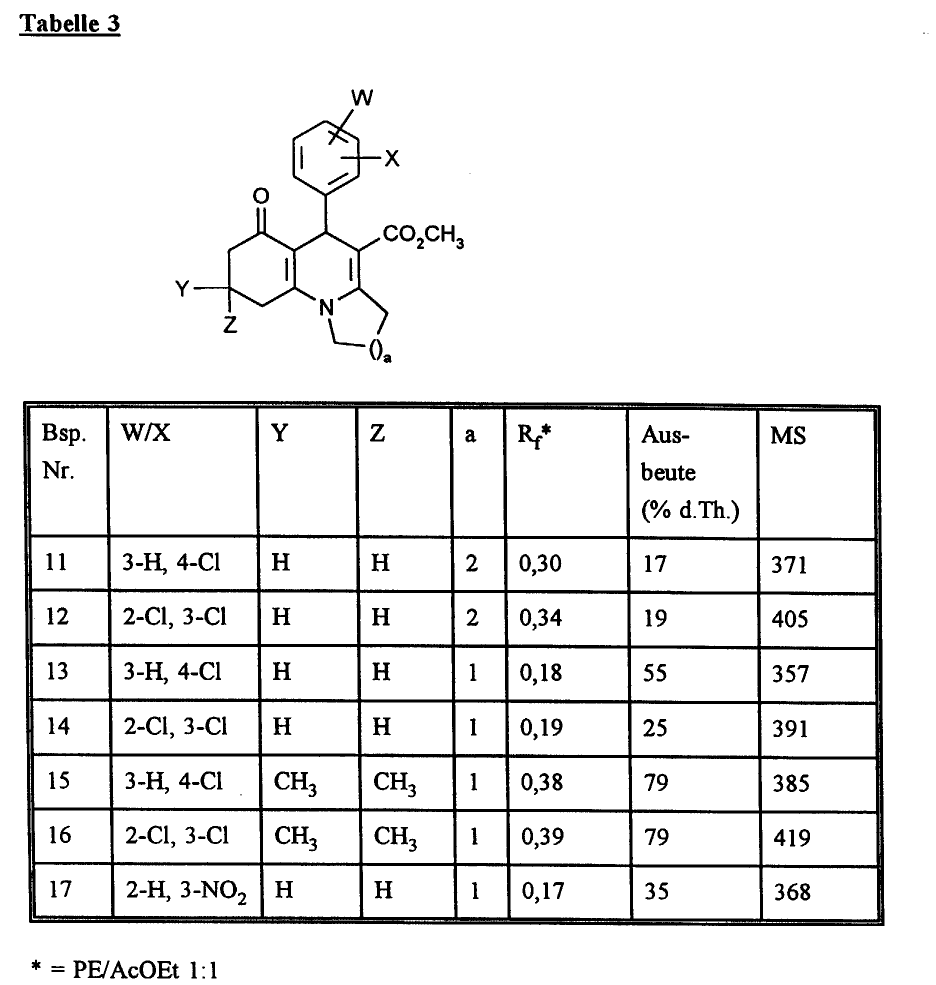

### Beispiel 18

6-Acetyl-7-(2,3-dichlorphenyl)-5-methyl-1,2,3,7-tetrahydroindolizin-8-carbonsäuremethylester 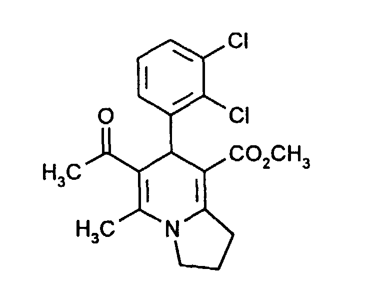 2,57 g (10 mmol) der Verbindung aus Beispiel A werden mit 1,4 g (10 mmol) Pyrrolidin-2-yliden-essigsäuremethylester in Pyridin gelöst und 12 h bei 100°C gehalten. Die Reaktionslösung wird abgekühlt, zweimal mit Toluol kodestilliert und der Rückstand durch Flashchromatographie gereinigt (Petrolether/AcOEt = 8:1). Die Titelverbindung kristallisiert aus Et₂O. Man erhält 250 mg Ausbeute (7 %).
R_{f} = 0,50 (PE/AcOEt = 1:1)
MS: 379

In Analogie zur Vorschrift des Beispiels 18 wird die in der Tabelle 4 aufgeführte Verbindung hergestellt:

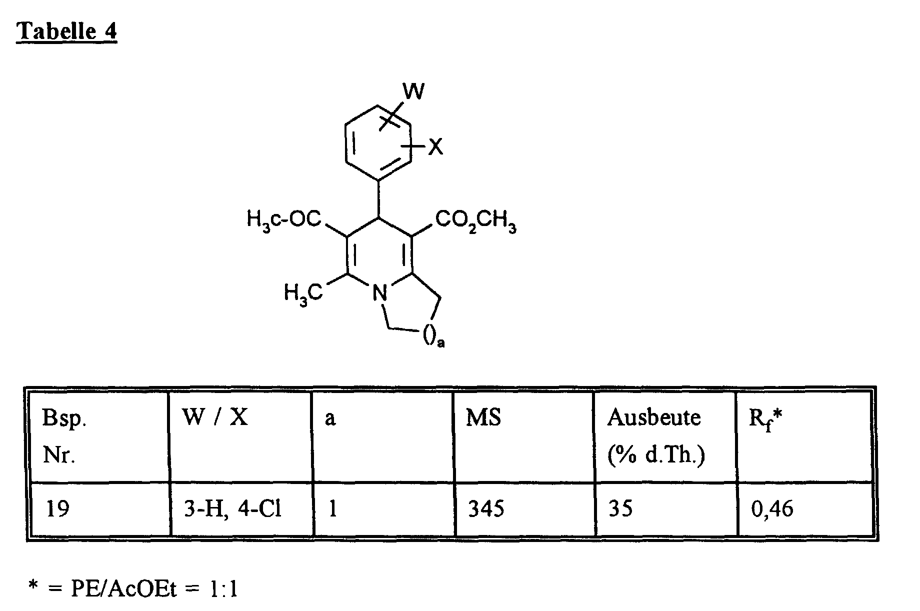

## Patentansprüche

1. Verbindungen, ausgewählt aus der folgenden Gruppe:
7-(2,3-Dichlorphenyl)-5-methyl-1,2,3,7-tetrahydroindolizin-6,8-dicarbonsäuredimethylester
5-Methyl-7-(4-trifluormethylphenyl)-1,2,3,7-tetrahyroindolizin-6,8-dicarbonsäuredimethylester
7-(4-Chlorphenyl)-5-methyl-1,2,3,7-tetrahydroindolicin-6,8-dicarbonsäuredimethylester
2-(4-Chlorphenyl)-4-methyl-2,6,7,8,9-pentahydro-quinolizin-1,3-dicarbonsäuredimethylester
1-[6-Acetyl-5-methyl-7-(4-nitrophenyl)-1,2,3,7-tetrahydroindolizin-8-yl]-ethanon
1-[6-Acetyl-7-(3,4-dichlorphenyl)-5-methyl-1,2,3,7-tetrahydroindolizin-8-yl]-ethanon
1-[6-Acetyl-7-(2,3-dichlorphenyl)-5-methyl-1,2,3,7-tetrahydroindolizin-8-yl]-ethanon
1-[6-Acetyl-5-methyl-7-(4-trifluormethylphenyl)-1,2,3,7-tetrahydroindolizin-8-yl]ethanon
1-[6-Acetyl-7-(4-chlor-3-trifluormethylphenyl)-5-methyl-1,2,3,7-tetrahydroindolizin-8-yl]ethanon
6-(3-Nitrophenyl)-7-oxo-1,2,3,4,6,7,8,9,10-nonahydro-pyrido[1,2-a]chinolin-5-carbonsäuremethylester
6-(4-Chlorphenyl)-7-oxo-1,2,3,4,6,7,8,9,10-nonahydro-pyrido[1,2-a]chinolin-5-carbonsäuremethylester
6-(2,3-Dichlorphenyl)-7-oxo-1,2,3,4,6,7,8,9,10-nonahydro-pyrido[1,2,a]chinolin-5-carbonsäuremethylester
5-(4-Chlorphenyl)-6-oxo-1,2,3,5,6,7,8,9-octahydropyrrolo[1,2-a]chinolin-4-carbonsäuremethylester
5-(2,3-Dichlorphenyl)-6-oxo-1,2,3,5,6,7,8,9-octahydropyrrolo[1,2-a]chinolin-4-carbonsäuremethylester
5-(4-Chlorphenyl)-8,8-dimethyl-6-oxo-1,2,3,5,6,7,8,9-octahydropyrrolo[1,2-a]chinolin-4-carbonsäuremethylester
5-(2,3-Dichlorphenyl)-8,8-dimethyl-6-oxo-1,2,3,5,6,7,8,9-octahydropyrrolo[1,2-a]chinolin-4-carbonsäuremethylester
5-(3-Nitrophenyl)-6-oxo-1,2,3,5,6,7,8,9-octahydropyrrolo[1,2-a]chinolin-4-carbonsäuremethylester
6-Acetyl-7-(2,3-dichlorphenyl)-5-methyl-1,2,3,7-tetrahydroindolizin-8-carbonsäuremethylester
6-Acetyl-7-(4-chlorphenyl)-5 -methyl-1,2,3,7-tetrahydroindolizin-8-carbonsäuremethylester.

2. Verfahren zur Herstellung von Verbindungen der allgemeinen Formel (I) 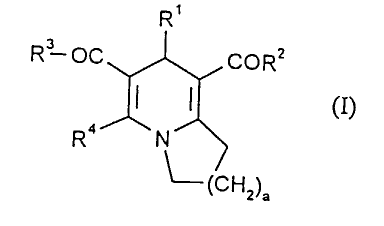 in welcher R¹, R², a, R³ und R⁴ die in der folgenden Tabelle angegebenen Substituentenbedeutungen haben,
| R¹ | R² | a | R³ | R³+R⁴ | R⁴ |
|---|---|---|---|---|---|
| m-NO₂-C₆H₄ | OCH₃ | 2 | - | -CH₂-CH₂-CH₂- | - |
| p-Cl-C₆H₄ | OCH₃ | 2 | - | -CH₂-CH₂-CH₂- | - |
| o,m-Cl-C₆H₃ | OCH₃ | 2 | - | -CH₂-CH₂-CH₂- | - |
| p-Cl-C₆H₄ | OCH₃ | 1 | - | -CH₂-CH₂-CH₂- | - |
| o,m-Cl-C₆H₃ | OCH₃ | 1 | - | -CH₂-CH₂-CH₂- | - |
| p-Cl-C₆H₄ | OCH₃ | 1 | - | -CH₂-C(CH₃)₂-CH₂- | - |
| o,m-Cl-C₆H₃ | OCH₃ | 1 | - | -CH₂-C(CH₃)₂-CH₂- | - |
| m-NO₂-C₆H₄ | OCH₃ | 1 | - | -CH₂-CH₂ -CH₂- | - |
| o,m-Cl-C₆H₃ | OCH₃ | 1 | -CH₃ | - | CH₃ |
| p-Cl-C₆H₄ | OCH₃ | 1 | -CH₃ | - | CH₃ |
| p-NO₂-C₆H₄ | CH₃ | 1 | CH₃ | - | CH₃ |
| m,p-Cl-C₆H₃ | CH₃ | 1 | CH₃ | - | CH₃ |
| o,m-Cl-C₆H₃ | CH₃ | 1 | CH₃ | - | CH₃ |
| p-CF₃-C₆H₄ | CH₃ | 1 | CH₃ | - | CH₃ |
| m-CF₃,p-Cl-C₆H₃ | CH₃ | 1 | CH₃ | - | CH₃ |
| o,m-Cl-C₆H₃ | OCH₃ | 1 | OCH₃ | - | CH₃ |
| p-CF₃-C₆H₄ | OCH₃ | 1 | OCH₃ | - | CH₃ |
| p-Cl-C₆H₄ | OCH₃ | 2 | OCH₃ | - | CH₃ |
| p-Cl-C₆H₄ | OCH₃ | 1 | OCH₃ | - | CH₃ |
dadurch gekennzeichnet, daß man
(A) im Fall, daß R³ und R⁴ jeweils für einen offenkettigen Rest stehen, Aldehyde der allgemeinen Formel (II)
R¹-CHO (II)
in welcher
R¹ die oben angegebene Bedeutung hat,
zunächst durch Umsetzung mit Dioxoverbindungen der allgemeinen Formel (III) 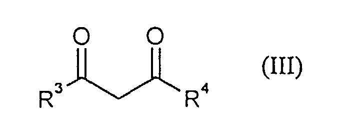 in welcher
R³ und R⁴ die oben angegebene Bedeutung haben,
in inerten Lösemitteln, gegebenenfalls in Anwesenheit einer Base,
in die Benzylidenverbindungen der allgemeinen Formel (IV) 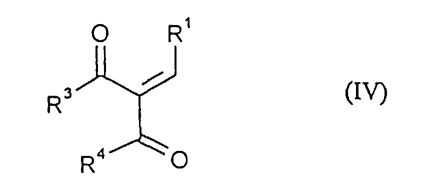 in welcher
R¹, R³ und R⁴ die oben angegebene Bedeutung haben,
überführt, welche gegebenenfalls isoliert werden,
und in einem zweiten Schritt mit Aminen der allgemeinen Formel (V) 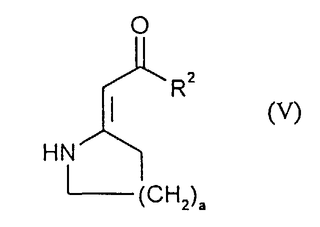 in welcher
R² und a die oben angegebene Bedeutung haben,
in inerten Lösemitteln umsetzt, oder
(B) im Fall, daß R³ und R⁴ gemeinsam einen der oben aufgeführten Ringe bilden,
die Aldehyde der allgemeinen Formel (II) mit Verbindungen der allgemeinen Formel (VI) 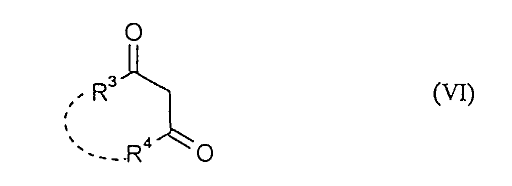 in welcher
R³ und R⁴ gemeinsam für einen Rest der Formel -CH₂-CH₂-CH₂- oder -CH₂-C(CH₃)₂-CH₂ stehen,
und den cyclischen Aminen der allgemeinen Formel (V), in einem Lösemittel, gegebenenfalls in Anwesenheit einer Base umsetzt.

3. Arzneimittel zur Behandlung des zentralen Nervensystems, enthaltend mindestens eine Verbindung der allgemeinen Formel (I) gemäß Anspruch 1 als selektiven Kaliumkanalmodulator.

4. Verfahren zur Herstellung von Arzneimitteln, dadurch gekennzeichnet, daß man mindestens eine Verbindung der allgemeinen Formel (I) gemäß Anspruch 1 gegebenenfalls unter Verwendung von üblichen Hilfs- und Trägerstoffen in eine geeignete Applikationsform überführt.

## Claims

1. Compounds selected from the following group:
Dimethyl 7-(2,3-dichlorophenyl)-5-methyl-1,2,3,7-tetrahydroindolizine-6,8-dicarboxylate
Dimethyl 5-methyl-7-(4-trifluoromethylphenyl)-1,2,3,7-tetrahydroindolizine-6,8-dicarboxylate
Dimethyl 7-(4-chlorophenyl)-5-methyl-1,2,3,7-tetrahydroindolizine-6,8-dicarboxylate
Dimethyl 2-(4-chlorophenyl)-4-methyl-2,6,7,8,9-pentahydro-quinolizine-1,3-dicarboxylate
1-[6-Acetyl-5-methyl-7-(4-nitrophenyl)- 1 ,2,3,7-tetrahydroindolizin-8-yl]ethanone
1-[6-Acetyl-7-(3,4-dichlorophenyl)-5-methyl- 1,2,3,7-tetrahydroindolizin-8-yl]ethanone
1-[6-Acetyl-7-(2,3-dichlorophenyl)-5-methyl-1,2,3,7-tetrahydroindolizin-8-yl]ethanone
1-[6-Acetyl-5-methyl-7-(4-trifluoromethylphenyl)-1,2,3,7-tetrahydroindolizin-8-yl]ethanone
1-[6-Acetyl-7-(4-chloro-3-trifluoromethylphenyl)-5-methyl-1,2,3,7-tetrahydroindolizin-8-yl]ethanone
Methyl 6-(3-nitrophenyl)-7-oxo-1,2,3,4,6,7,8,9,10-nonahydropyrido[1,2-a]quinoline-5-carboxylate
Methyl 6-(4-chlorophenyl)-7-oxo-1,2,3,4,6,7,8,9,10-nonahydropyrido[1,2-a]quinoline-5-carboxylate
Methyl 6-(2,3-dichlorophenyl)-7-oxo-1,2,3,4,6,7,8,9,10-nonahydropyrido[1,2-a]quinoline-3-carboxylate
Methyl 5-(4chlorophenyl)-6-oxo-1,2,3,5,6,7,8,9-octahydro-pyrrolo-[1,2-a]quinoline-4-carboxylate
Methyl 5-(2,3-dichlorophenyl)-6-oxo-1,2,3,5,6,7,8,9-octahydropyrrdo[1,2-a]quinoline-4-carboxylate
Methyl 5-(4-chlorophenyl)-8,8-dimethyl-6-oxo-1,2,3,5,6,7,8,9-octahydropyrrolo[1,2-a]quinoline-4-carboxylate
Methyl 5-(2,3-dichlorophenyl)-8,8-dimethyl-6-oxo-1,2,3,5,6,7,8,9-octahydropyrrolo[1 ,2-a]quinoline-4-carboxylate
Methyl 5-(3-nitrophenyl)-6-oxo-1,2,3,5,6,7,8,9-octahydropyrrolo[1,2-a]quinoline-4-carboxylate
Methyl 6-acetyl-7-(2,3,-dichlorophenyl)-5-methyl-1,2,3,7-tetrahydroindolizine-8-carboxylate
Methyl 6-acetyl-7-(4-chlorophenyl)5-methyl-1,2,3,7-tetrahydroindolizine-8-carboxylate.

2. Process for the preparation of compounds of the general formula (I) 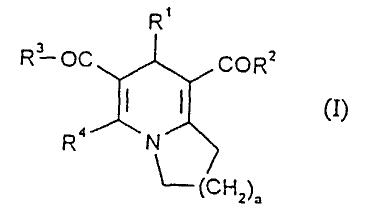 in which R¹, R², a, R³ and R⁴ have the substituent meanings given in the following table,
| R¹ | R² | a | R³ | R³+R⁴ | R4 |
|---|---|---|---|---|---|
| m-NO₂-C₆H₄ | OCH₃ | 2 | - | -CH₂-CH₂-CH₂- | - |
| p-Cl-C₆H₄ | OCH₃ | 2 - | - | -CH₂-CH₂-CH₂- | - |
| o,m-Cl-C₆H₃ | OCH₃ | 2 | - | -CH₂-CH₂-CH₂- | - |
| p-Cl-C₆H₄ | OCH₃ | 1 | - | -CH₂-CH₂-CH₂- | - |
| o,m-Cl-C₆H₃ | OCH₃ | 1 | - | -CH₂-CH₂-CH₂- | - |
| p-Cl-C₆H₄ | OCH₃ | 1 | - | -CH₂-C(CH₃)₂-CH₂- | - |
| o,m-Cl-C₆H₃ | OCH₃ | 1 | - | -CH₂-C(CH₃)₂-CH₂- | - |
| m-NO₂-C₆H₄ | OCH₃ | 1 | - | -CH₂-C-CH₂- | - |
| o,m-Cl-C₆H₃ | OCH₃ | 1 | -CH₃ | - | CH₃ |
| p-Cl-C₆H₄ | OCH₃ | 1 | -CH₃ | - | CH₃ |
| p-NO₂-C₆H₄ | CH₃ | 1 | CH₃ | - | CH₃ |
| m,p-Cl-C₆H₃ | CH₃ | 1 | CH₃ | - | CH₃ |
| o,m-Cl-C₆H₃ | CH₃ | 1 | CH₃ | - | CH₃ |
| p-CF₃-C₆H₄ | CH₃ | 1 | CH₃ | - | CH₃ |
| m-CF3, p-Cl-C₆H₃ | CH₃ | 1 | CH₃ | - | CH₃ |
| o,m-Cl-C₆H₃ | OCH₃ | 1 | OCH₃ | - | CH₃ |
| p-CF₃-C₆H₄ | OCH₃ | 1 | OCH₃ | - | CH₃ |
| p-Cl-C₆H₄ | OCH₃ | 2 | OCH₃ | - | CH₃ |
| p-Cl-C₆H₄ | OCH₃ | 1 | OCH₃ | - | CH₃ |
characterized in that
(A) in the case in which R³ and R⁴ each represent an open-chain radical, aldehydes of the general formula (II)
R¹-CHO (II)
in which
R¹ has the meaning indicated above,
are first converted by reaction with dioxo compounds of the general formula (III) 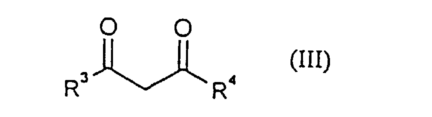 in which
R³ and R⁴ have the meaning indicated above,
in inert solvents, if appropriate in the presence of a base,
into the benzylidene compounds of the general formula (IV) 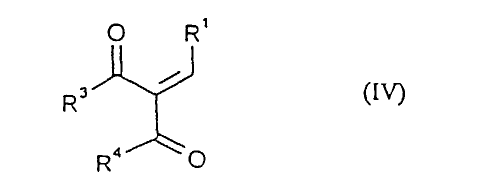 in which
R¹, R³ and R⁴ have the meaning indicated above,
which are optionally isolated,
and in a second step with amines of the general formula (V) 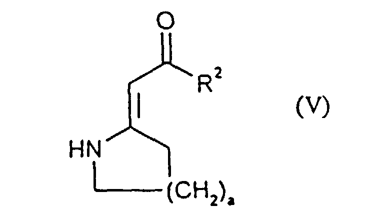 in which
R² and a have the meaning indicated above,
in inert solvents, or
(B) in the case in which R³ and R⁴ together form one of the abovementioned rings,
the aldehydes of the general formula (II) are reacted with compounds of the general formula (VI) 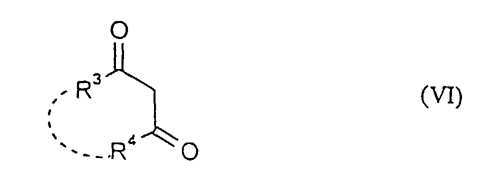 in which
R³ and R⁴ together represent a radical of the formula -CH₂-CH₂-CH₂- or -CH₂-C(CH₃)₂-CH₂-,
and the cyclic amines of the general formula (V), in a solvent, if appropriate in the presence of a base.

3. Medicaments for the treatment of the central nervous system, containing at least one compound of the general formula (I) according to Claim 1 as a selective potassium channel modulator.

4. Process for the production of medicaments, characterized in that at least one compound of the general formula (I) according to Claim 1 is converted into a suitable application form, if appropriate using customary auxiliaries and excipients.

## Revendications

1. Composés choisis dans le groupe suivant :
ester de diméthyle d'acide 7-(2,3-dichlorophényl)-5-méthyl-1,2,3,7-tétrahydro-indolizine-6,8-dicarboxylique,
ester de diméthyle d'acide 5-méthyl-7-(4-trifluorométhylphényl)-1,2,3,7-tétrahydro-indolizine-6,8-dicarboxylique,
ester de diméthyle d'acide 7-(4-chlorophényl)-5-méthyl-1,2,3,7-tétrahydro-indolizine-6,8-dicarboxylique,
ester de diméthyle d'acide 2-(4-chlorophényl)-4-méthyl-2,6,7,8,9-pentahydro-quinolizine-1,3-dicarboxylique,
1-[6-(acétyl-5-méthyl-7-(4-nitrophényl)-1,2,3,7-tétrahydroindolizine-8-yl]éthanone,
1-[6-(acétyl-7-(3,4-dichlorophényl)-5-méthyl-1,2,3,7-tétrahydro-indolizine-8-yl]éthanone,
1-[6-(acétyl-7-(2,3-dichlorophényl)-5-méthyl-1,2,3,7-tétrahydro-indolizine-8-yl]éthanone,
1-[6-(acétyl-5-méthyl-7-(4-trifluorométhylphényl)-1,2,3,7-tétrahydro-indolizine-8-yl]éthanone,
1-[6-(acétyl-7-(4-chloro-3-trifluorométhylphényl)-5-méthyl-1,2,3,7-tétrahydro-indolizine-8-yl]éthanone,
ester de méthyle d'acide 6-(3-nitrophényl)-7-oxo-1,2,3,4,6,7,9,10-nonahydro-pyrido[l,2-a]quinoline-5-carboxylique,
ester de méthyle d'acide 6-(4-chlorophényl)-7-oxo-1,2,3,4,6,7,8,9,10-nonahydro-pyrido[1,2-a]quinoline-5-carboxylique,
ester de méthyle d'acide 6-(2,3-dichlorophényl)-7-oxo-1,2,3,4,6,7,8,9,10-nonahydro-pyrido[1,2-a]quinoline-5-carboxylique,
ester de méthyle d'acide 5-(4-chlorophényl)-6-oxo-1,2,3,5,6,7,8,9-octahydropyrrolo[1,2-a]quinoline-4-carboxylique,
ester de méthyle d'acide 5-(2,3-dichlorophényl)-6-oxo-1,2,3,5,6,7,8,9-octahydropyrrolo[1,2-a]quinoline-4-carboxylique,
ester de méthyle d'acide 5-(4-chlorophényl)-8,8-diméthyl-6-oxo-1,2,3,5,6,7,8,9-octahydropyrrolo[1,2-a]quinoline-4-carboxylique,
ester de méthyle d'acide 5- (2,3-dichlorophényl)-8,8-diméthyl-6-oxo-1,2,3,5,6,7,8,9-octahydropyrrolo[1,2-a]quinoline-4-carboxylique,
ester de méthyle d'acide 5-(3-nitrophényl)-6-oxo-1,2,3,5,6,7,8,9-octahydropyrrolo[1,2-a]quinoline-4-carboxylique,
ester de méthyle d'acide 6-acétyl-7-(2,3-dichlorophényl)-5-méthyl-1,2,3,7-tétrahydro-indolizine-8-carboxylique,
ester de méthyle d'acide 6-acétyl-7-(4-chlorophényl)-5-méthyl-1,2,3,7-tétrahydro-indolizine-8-carboxylique.

2. Procédé de production de composés de formule générale (I) 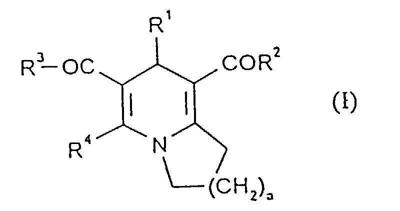 dans laquelle R¹, R², a, R³ et R⁴ ont les définitions de substituants indiquées dans le tableau ci-après,
| R¹ | R² | a | R³ | R³+R⁴ | R⁴ |
|---|---|---|---|---|---|
| m-NO₂-C₆H₄ | OCH₃ | 2 | - | -CH₂-CH₂-CH₂- | - |
| p-Cl-C₆H₄ | OCH₃ | 2 | - | -CH₂-CH₂-CH₂- | - |
| o,m-Cl-C₆H₃ | OCH₃ | 2 | - | -CH₂-CH₂-CH₂- | - |
| p-Cl-C₆H₄ - | OCH₃ | 1 | - | -CH₂-CH₂-CH₂- | - |
| o,m-Cl-C₆H₃ | OCH₃ | 1 | - | -CH₂-CH₂-CH₂- | - |
| p-Cl-C₆H₄ | OCH₃ | 1 | - | -CH₂-C(CH₃)₂-CH₂- | - |
| o,m-Cl-C₆H₃ | OCH₃ | 1 | - | -CH₂-C(CH₃)₂-CH₂- | - |
| m-NO₂-C₆H₄ | OCH₃ | 1 | - | -CH₂-C-CH₂- | - |
| o,m-Cl-C₆H₃ | OCH₃ | 1 | -CH₃ | - | CH₃ |
| p-Cl-C₆H₄ | OCH₃ | 1 | -CH₃ | - | CH₃ |
| p-NO₂-C₆H₄ | CH₃ | 1 | CH₃ | - | CH₃ |
| m,p-Cl-C₆H₃ | CH₃ | 1 | CH₃ | - | CH₃ |
| o,m-Cl-C₆H₃ | CH₃ | 1 | CH₃ | - | CH₃ |
| p-CF₃-C₆H₄ | CH₃ | 1 | CH₃ | - | CH₃ |
| m-CF₃,p-Cl-C₆H₃ | CH₃ | 1 | CH₃ | - | CH₃ |
| o,m-Cl-C₆H₃ | OCH₃ | 1 | OCH₃ | - | CH₃ |
| p-CF₃-C₆H₄ | OCH₃ | 1 | OCH₃ | - | CH₃ |
| p-Cl-C₆H₄ | OCH₃ | 2 | OCH₃ | - | CH₃ |
| p-Cl-C₆H₄ | OCH₃ | 1 | OCH₃ | - | CH₃ |
caractérisé en ce que
(A) au cas où R³ et R⁴ représentent chacun un reste à chaîne ouverte, on transforme des aldéhydes de formule générale (II)
R¹-CHO (II)
dans laquelle
R¹ a la définition indiquée ci-dessus, tout d'abord par réaction avec des composés dioxo de formule générale (III) 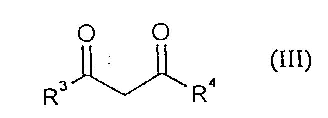 dans laquelle
R³ et R⁴ ont la définition indiquée ci-dessus,
dans des solvants inertes, le cas échéant en présence d'une base,
en composés benzylidéniques de formule générale (IV) 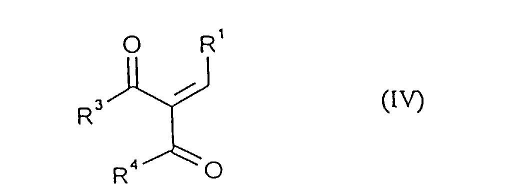 dans laquelle
R¹, R³ et R⁴ ont la définition indiquée ci-dessus,
que l'on isole le cas échéant,
et dans une seconde étape, on fait réagir ces composés avec des amines de formule générale (V) 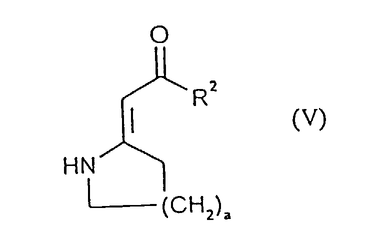 dans laquelle
R² et a ont la définition indiquée ci-dessus,
dans des solvants inertes, ou bien
(B) au cas où R³ et R⁴ forment ensemble l'un des noyaux indiqués ci-dessus,
on fait réagir les aldéhydes de formule générale (II) avec des composés de formule générale (VI) 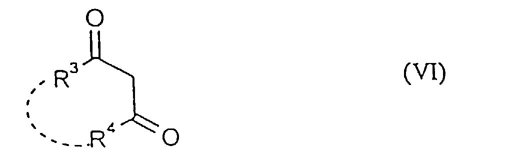 dans laquelle
R³ et R⁴ forment un reste de formule -CH₂-CH₂-CH₂- ou -CH₂C(CH₃)₂-CH₂,
et les amines cycliques de formule générale (V) dans un solvant, le cas échéant en présence d'une base.

3. Médicament destiné au traitement du système nerveux central, comprenant au moins un composé de formule générale (I) suivant la revendication 1 comme modulateur sélectif des canaux potassium.

4. Procédé de préparation de médicaments, caractérisé en ce qu'on fait prendre à un composé de formule générale (I) suivant la revendication 1 une forme d'administration appropriée, le cas échéant en utilisant des substances auxiliaires et des supports classiques.
